(19) Europäisches Patentamt
European Patent Office
Office européen des brevets

(11) Veröffentlichungsnummer: **0 296 452**
**A1**

(12) # EUROPÄISCHE PATENTANMELDUNG

(21) Anmeldenummer: 88109423.9

(22) Anmeldetag: 14.06.88

(51) Int. Cl.⁴: C07D 249/08 , C07D 233/60 , C07D 303/34 , C07C 149/267 , C07C 149/273 , A01N 43/50 , A01N 43/653

(30) Priorität: 24.06.87 DE 3720756

(43) Veröffentlichungstag der Anmeldung: 28.12.88 Patentblatt 88/52

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Stroech, Klaus, Dr.
Rolsberger Strasse 22
D-5650 Solingen 1(DE)
Erfinder: Bielefeldt, Dietmar, Dr.
Beuthener Strasse 13
D-4030 Ratingen 6(DE)
Erfinder: Brandes, Wilhelm, Dr.
Eichendorffstrasse 3
D-5653 Leichlingen(DE)
Erfinder: Dutzmann, Stefan, Dr.
Leinenweberweg 33
D-4000 Düsseldorf 13(DE)

(54) **Azolylmethyl-cyclopropyl-carbinol-Derivate.**

(57) Azolylmethyl-cyclopropyl-carbinol-Derivate der Formel I

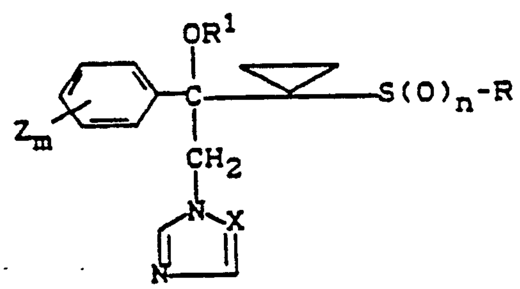

(I)

in welcher
R für Perfluoralkyl mit 1 bis 4 Kohlenstoffatomen, Trichlormethyl, Difluorchlormethyl oder Fluordichlormethyl steht,
$R^1$ für Wasserstoff, Alkyl oder Acyl steht,
Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen,

EP 0 296 452 A1

Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,

m für die Zahlen 0, 1, 2 oder 3 steht und

n für die Zahlen 0, 1 oder 2 steht und

X für Stickstoff oder eine CH-Gruppe steht,

sowie deren Säureadditions-Salze und Metallsalz-Komplexe,

mehrere Verfahren zur Herstellung der neuen Stoffe und deren Verwendung als Fungizide und Pflanzenwachstumsregulatoren.

Neue Zwischenprodukte, verfahren zu deren Herstellung und deren Verwendung zur Synthese der neuen Stoffe der Formel (I).

## Azolylmethyl-cyclopropyl-carbinol-Derivate

Die vorliegende Erfindung betrifft neue Azolylmethylcyclopropyl-carbinol-Derivate, mehrere Verfahren zu deren Herstellung und deren Verwendung als Fungizide und Pflanzenwachstrumsregulatoren.

Es ist bereits bekannt geworden, daß bestimmte Azolylmethyl-cyclopropyl-carbinol-Derivate fungizide und pflanzenwuchsregulierende Eigenschaften besitzen (vgl. EP-OS 0 180 136 und DE-OS 3 617 190). So kann zum Beispiel 1-(4-Chlorphenyl)-1-[1-(methylthio)-1-cyclopropyl]-2-(1,2,4-triazol-1-yl)-ethan-1-ol zur Bekämpfung von Pilzen und zur Regulierung des Pflanzenwachstums eingesetzt werden. Die Wirksamkeit dieses Stoffes ist gut; sie läßt allerdings bei niedrigen Aufwandmengen in manchen Fällen zu wünschen übrig.

Es wurden nun neue Azolylmethyl-cyclopropyl-carbinol-Derivate der Formel

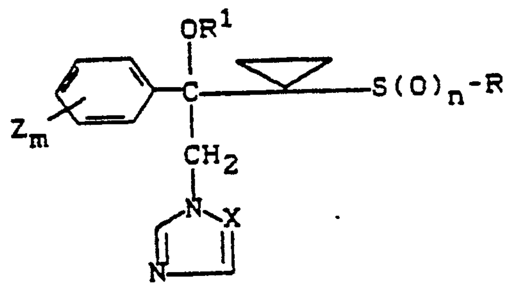

(I)

in welcher

R für Perfluoralkyl mit 1 bis 4 Kohlenstoffatomen, Trichlormethyl, Difluorchlormethyl oder Fluordichlormethyl steht,

$R^1$ für Wasserstoff, Alkyl oder Acyl steht,

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,

m für die Zahlen 0, 1, 2 oder 3 steht und

n für die Zahlen 0, 1 oder 2 steht und

X für Stickstoff oder eine CH-Gruppe steht,

sowie deren Säureadditions-Salze und Metallsalz-Komplexe gefunden.

Weiterhin wurde gefunden, daß man Azolylmethyl-cyclopropyl-carbinol-Derivate der Formel (I) sowie deren Säureadditions-Salze and Metallsalz-Komplexe erhält, wenn man

a) in einer <u>ersten Stufe</u> Phenyl-cyclopropyl-ketone der Formel

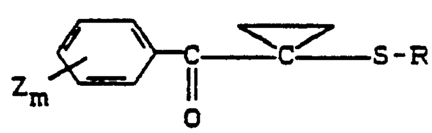

(II)

in welcher

R, Z und m die oben angegebene Bedeutung haben,

entweder

α) mit Dimethyloxosulfonium-methylid der Formel

$$\overset{\delta\oplus}{(CH_3)_2SO}\overset{\delta\ominus}{CH_2} \qquad (III)$$

oder

β) mit Dimethylsulfonium-methylid der Formel

$$(CH_3)_2\overset{\delta\oplus}{S}\;\overset{\delta\ominus}{CH_2} \qquad (IV)$$

in Gegenwart eines Verdünnungsmittels umsetzt und in einer zweiten Stufe die dabei erhaltenen Oxirane der Formel

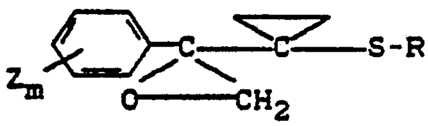

(V)

in welcher
R, Z und m die oben angegebene Bedeutung haben,
mig Azolen der Formel

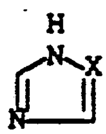

(VI)

in welcher
X die oben angegebene Bedeutung hat,
in Gegenwart eines Säurebindemittels und in Gegenwart eines Verdünnungsmittels umsetzt,
oder

b) Azolylmethyl-cyclopropyl-carbinol-Derivate der Formel

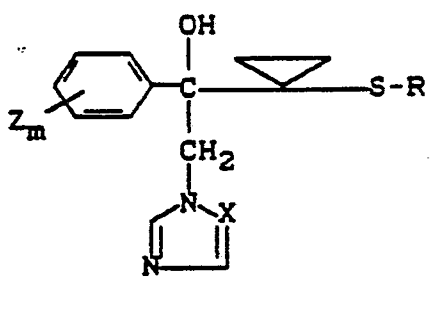

(Ia)

in welcher
R, X, Z und m die oben angegebenen Bedeutung haben,
mit Oxidationsmitteln gegebenenfalls in Gegenwart eines Verdünnungsmittels umsetzt,
oder

c) Azolylmethyl-cyclopropyl-carbinol-Derivate der Formel

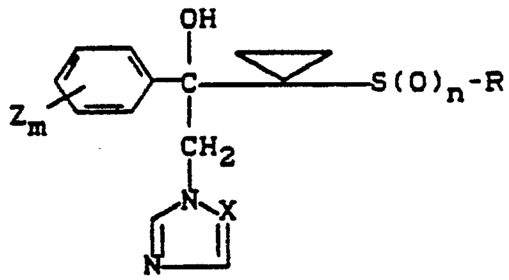

(Ib)

in welcher
R, Z, X, m und n die oben angegebene Bedeutung haben,
mit starken Basen in Gegenwart eines Verdünnungsmittels umsetzt und die dabei entstehenden Alkoholate der Formel

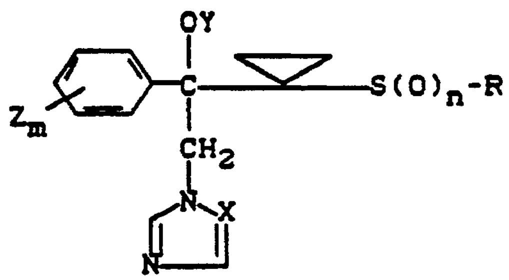

(Ic)

in welcher
R, X, Z, m und n die oben angegebene Bedeutung haben und
Y für einen kationischen Rest einer Base steht, mit Halogenverbindungen der Formel

$R^2$-Hal (VII)

in welcher
$R^2$ für Alkyl oder Acyl steht und
Hal für Halogen steht,
in Gegenwart eines Verdünnungsmittels umsetzt, und gegebenenfalls anschließend an die so erhaltenen Verbindungen der Formel (I) eine Säure oder ein Metallsalz addiert.

Schließlich wurde gefunden, daß die neuen Azolylmethylcyclopropyl-carbinol-Derivate der Formel (I) sowie deren Säureadditions-Salze und Metallsalz-Komplexe starke fungizide und pflanzenwuchsregulierende Eigenschaften besitzen.

Die erfindungsgemäßen Stoffe enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemische.

Überraschenderweise besitzen die erfindungsgemäßen Stoffe eine bessere fungizide und pflanzenwuchsregulierende Wirksamkeit als 1-(4-Chlorphenyl)-1-[1-(methylthio)-1-cyclopropyl]-2-(1,2,4-triazol-1-yl)-ethan-1-ol, welches der konstitutionell ähnlichste vorbekannte Wirkstoff gleicher Wirkungsrichtung ist.

Die erfindungsgemäßen Azolylmethyl-cyclopropyl-carbinol-Derivate sind durch die Formel (I) allgemein definiert. In dieser Formel stehen vorzugsweise
R für Trifluormethyl, Trichlormethyl, Difluorchlormethyl oder Fluordichlormethyl,
R' für Wasserstoff, Methyl, Ethyl, n-Propyl, Isopropyl, n-Butyl, Isobutyl, Methylcarbonyl, Ethylcarbonyl, n-Propylcarbonyl, Isopropyl-carbonyl, n-Butylcarbonyl und Isobutyl-carbonyl,
Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy,
m für die Zahlen 0, 1, 2 oder 3,
n für die Zahlen 0, 1 oder 2 und

X für Stickstoff oder eine CH-Gruppe.

Wenn m für die Zahlen 2 oder 3 steht, können die für Z stehenden Reste gleich oder verschieden sein.

Bevorzugte erfindungsgemäße Verbindungen sind auch Additionsprodukte aus Säuren und denjenigen Azolylmethylcyclopropyl-carbinol-Derivaten der Formel (I), in denen R, $R^1$, X, Z, m und n die Bedeutungen haben, die bereits vorzugsweise für diese Reste und Indices genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Azolylmethyl-cyclopropyl-carbinol-Derivaten der Formel (I), in denen R, $R^1$, X, Z, m und n die Bedeutungen haben, die bereits vorzugsweise für diese Reste und Indices genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für Azolylmethyl-cyclopropyl-carbinol-Derivate der Formel (I) seien die in der folgenden

Tabelle aufgeführten Stoffe genannt.

**Tabelle**

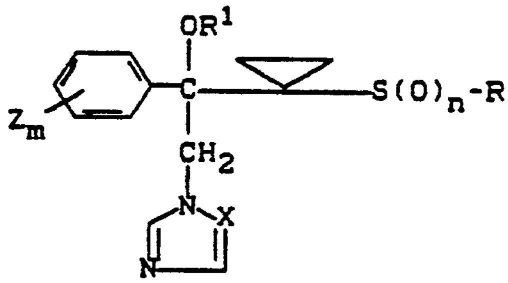

(I)

| $Z_m$ | R | $R^1$ | X | n |
|---|---|---|---|---|
| 4-Cl | $CF_3$ | H | N | 0 |
| 2,4-$Cl_2$ | $CF_3$ | H | N | 0 |
| 2,4-$F_2$ | $CF_3$ | H | N | 0 |
| 4-Cl | $CF_3$ | H | CH | 0 |
| 4-Cl | $CF_3$ | H | CH | 1 |
| 4-Cl | $CF_3$ | H | CH | 2 |
| 4-Cl | $CF_3$ | $CH_3$ | N | 0 |
| 4-$CH_3$ | $CF_3$ | H | N | 0 |
| 4-$CF_3$ | $CF_3$ | H | N | 0 |
| 4-$OCF_3$ | $CF_3$ | H | N | 0 |
| 4-$SCF_3$ | $CF_3$ | H | N | 0 |
| 4-$OCH_3$ | $CF_3$ | H | N | 0 |
| 4-$SCH_3$ | $CF_3$ | H | N | 0 |
| 2,4,6-$Cl_3$ | $CF_3$ | H | N | 0 |

Tabelle (Fortsetzung)

| $Z_m$ | R | $R^1$ | X | n |
|---|---|---|---|---|
| 4- 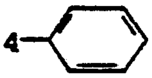 | $CF_3$ | H | N | 0 |
| 4-O 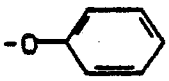 | $CF_3$ | H | N | 0 |
| $4\text{-}C_4H_9\text{-t.}$ | $CF_3$ | H | N | 0 |
| $2\text{-Cl,4-}CH_3$ | $CF_3$ | H | N | 0 |
| $2,4\text{-}Cl_2$ | $CF_3$ | H | N | 1 |
| $2,4\text{-}Cl_2$ | $CF_3$ | H | N | 2 |
| $2,4\text{-}Cl_2$ | $CF_3$ | $-CO-CH_3$ | N | 0 |
| $2,4\text{-}Cl_2$ | $CF_3$ | $-C_2H_5$ | N | 0 |
| $2,4\text{-}Cl_2$ | $CF_3$ | $-CH_3$ | N | 0 |
| $2,4\text{-}Cl_2$ | $CF_3$ | H | CH | 0 |
| - | $CF_3$ | H | N | 0 |
| - | $CF_3$ | H | CH | 0 |
| - | $CF_3$ | H | N | 1 |
| - | $CF_3$ | H | N | 2 |
| $2,4\text{-}F_2$ | $CF_3$ | H | CH | 0 |
| 4-F | $CF_3$ | H | CH | 0 |
| 4-F | $CF_3$ | $CH_3$ | N | 0 |
| 4-F | $CF_3$ | $CH_3$ | N | 2 |

Verwendet man 2,4-Difluorphenyl-1-(trifluormethylmercapto)-cycloprop-1-yl-keton und Dimethyloxosulfonium-methylid als Ausgangsstoffe und 1,2,4-Triazol als Reaktionskomponente, so kann der Verlauf des erfindungsgemäßen Verfahrens (a) durch das folgende Formelschema veranschaulicht werden:

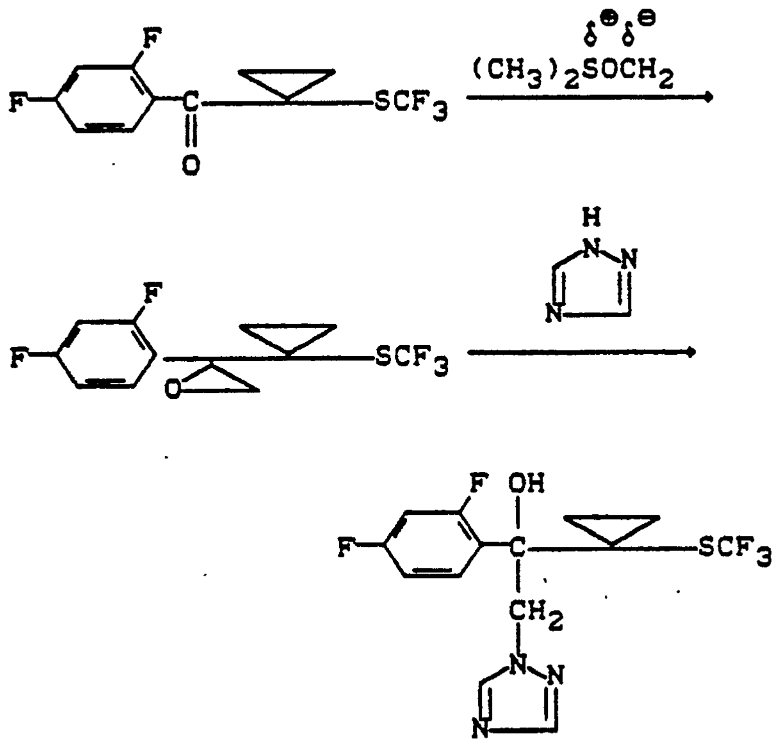


Verwendet man 1-(2,4-Difluorphenyl)-1-hydroxy-2-(1,2,4-triazol-1-yl)-1-(1-trifluormethylmercapto-cycloprop-1-yl)-ethan und Wasserstoffperoxid in Eisessig als Ausgangsstoffe, so kann der Verlauf des erfindungsgemäßen Verfahrens (b) durch das folgende Formelschema veranschaulicht werden:

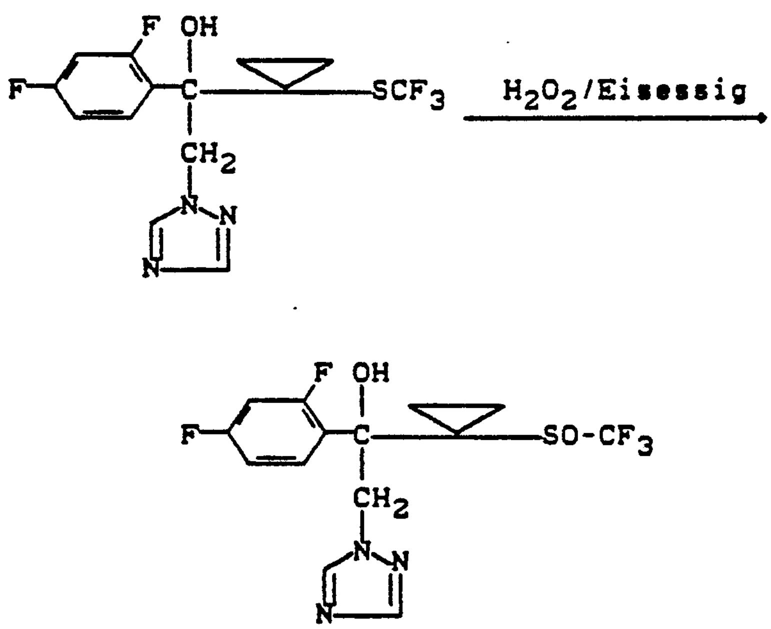


Verwendet man 1-(2,4-Difluorphenyl)-1-hydroxy-2-(1,2,4-triazol-1-yl)-1-(1-trifluormethylmercapto-cycloprop-1-yl)-ethan und Natriumhydrid als Ausgangsstoffe und Iodmethan als Reaktionskomponente, so kann der Verlauf des erfindungsgemäßen Verfahrens (c) durch das folgende Formelschema veranschaulicht werden:

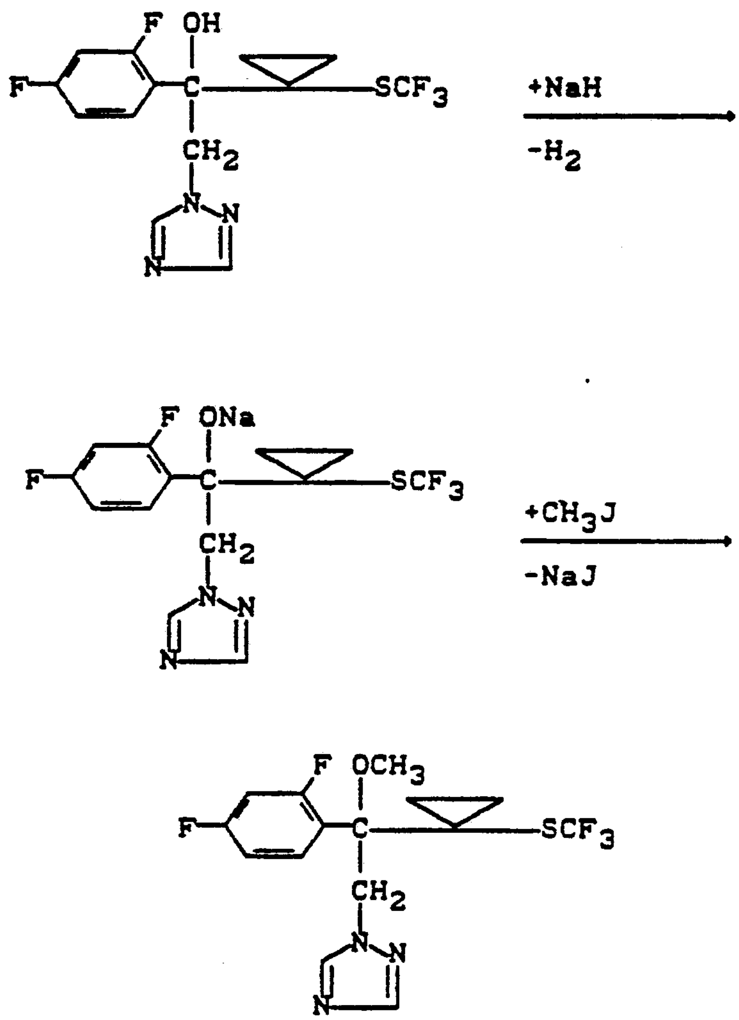


Die bei dem erfindungsgemäßen Verfahren (a) als Ausgangsstoffe benötigten Phenyl-cyclopropyl-ketone sind durch die Formel (II) allgemein definiert. In dieser Formel haben R, Z und m vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden.

Die Phenyl-cyclopropyl-ketone der Formel (II) sind bisher noch nicht bekannt. Sie lassen sich herstellen, indem man

d) in einer <u>ersten Stufe</u> Phenylketone der Formel

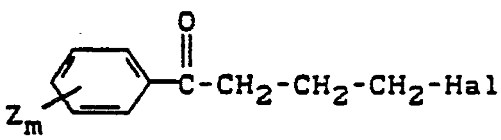
(VIII)

in welcher
Z und m die oben angegebene Bedeutung haben und
Hal für Chlor oder Brom steht,
mit Mercaptanen der Formel

Hal′-S-R (IX)

in welcher
R die oben angegebene Bedeutung hat und
Hal′ für Chlor oder Brom steht,

in Gegenwart eines Verdünnungsmittels umsetzt und die entstehenden Phenylpropyl-ketone der Formel

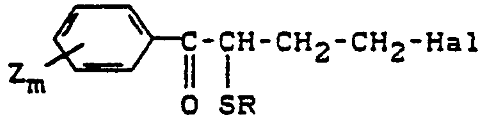

(X)

in welcher
R, Z, Hal und m die oben angegebene Bedeutung haben,
in einer zweiten Stufe in Gegenwart eines Säurebindemittels und in Gegenwart eines Verdünnungsmittels umsetzt.

Die bei dem Verfahren (d) als Ausgangsstoffe benötigten Phenylketone der Formel (VIII) sind bekannt oder lassen sich nach prinzipiell bekannten Verfahren in einfacher Weise herstellen (vgl. DE-OS 2 521 104, DE-OS 2 320 355 und DE-OS 2 351 948). So erhält man Phenylketone der Formel (VIII), indem man Benzol-Derivate der Formel

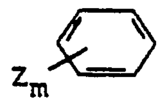

(XI)

in welcher
Z und m die oben angegebene Bedeutung haben,
mit Buttersäurehalogeniden der Formel

$$Cl-\overset{O}{\overset{\|}{C}}-CH_2-CH_2-CH_2-Hal \quad (XII)$$

in welcher
Hal die oben angegebene Bedeutung hat,
in Gegenwart eines Friedel-Crafts-Katalysators, wie zum Beispiel Aluminiumchlorid, gegebenenfalls in Gegenwart eines Verdünnungsmittels, wie zum Beispiel Methylenchlorid, bei Temperaturen zwischen -15 °C und +60 °C umsetzt.

Die bei dem Verfahren (d) weiterhin als Ausgangsstoffe benötigten Mercaptane der Formel (IX) sind bekannt.

Als Verdünnungsmittel kommen bei der Durchführung der ersten Stufe des Verfahrens (d) alle für derartige Umsetzungen üblichen inerten organischen Solventien in Betracht. Vorzugsweise verwendbar sind halogenierte aliphatische Kohlenwasserstoffe, wie zum Beispiel Methylenchlorid, Chloroform und Tetrachlorkohlenstoff.

Die Reaktionstemperaturen können bei der Durchführung der ersten Stufe des Verfahrens (d) in einem bestimmten Bereich variiert werden. Im allgemeinen arbeitet man bei Temperaturen zwischen 0 °C und 80 °C, vorzugsweise zwischen 10 und 50 °C.

Bei der Durchführung der ersten Stufe des Verfahrens (d) setzt man auf 1 Mol an Phenylketon der Formel (VIII) im allgemeinen 2 bis 4 Mol an Mercaptanen der Formel (IX) ein. Die Isolierung der entstehenden Phenyl-propylketone der Formel (X) erfolgt nach üblichen Methoden. Im allgemeinen geht man so vor, daß man das Lösungsmittel abzieht und das verbleibende Produkt entweder direkt oder nach vorheriger Reinigung für die weitere Synthese einsetzt.

Bei der Durchführung der zweiten Stufe des Verfahrens (d) kommen als Säurebindemittel alle für derartige Umsetzungen üblichen Basen in Betracht. Vorzugsweise verwendbar sind Alkoholate, wie Natriummethylat oder Kalium-tert.-butylat, ferner niedere tertiäre Alkylamine, Cycloalkylamine und Aryl-alkyl-amine, wie Triethylamin, Dimethyl-anilin, 1,5-Diaza-bicyclo[4.3.0]non-5-en und 1,8-Diazabicyclo[5.4.0]undecan.

Als Verdünnungsmittel kommen bei der Durchführung der zweiten Stufe des Verfahrens (d) alle für derartige Umsetzungen üblichen inerten organischen Solventien in Frage. Vorzugsweise verwendbar sind Alkohole, wie Methanol, Ethanol, Methoxyethanol, Propanol oder tert.-Butanol, ferner Ketone, wie Aceton und Butan-2-on, außerdem Nitrile, wie Acetonitril, weiterhin Ester, wie Essigester, darüber hinaus Ether, wie Dioxan, aromatische Kohlenwasserstoffe, wie Benzol oder Toluol, und auch Amide, wie Dimethylformamid.

Die Temperaturen können auch bei der Durchführung der zweiten Stufe des Verfahrens (d) in einem

größeren Bereich variiert werden. Im allgemeinen arbeitet man bei Temperaturen zwischen 0 °C und 100 °C, vorzugsweise zwischen 20 °C und 60 °C.

Bei der Durchführung der zweiten Stufe des Verfahrens (d) setzt man auf 1 Mol an Phenyl-propyl-keton der Formel (X) im allgemeinen 1 bis 3 Mol an Base ein. Die Aufarbeitung erfolgt nach üblichen Methoden. Im allgemeinen geht man so vor, daß man des Reaktionsgemisch mit Wasser vermengt, anschließend mit einem in Wasser wenig löslichen organischen Lösungsmittel extrahiert, die vereinigten organischen Phasen mit Wasser wäscht und nach dem Trocknen einengt. Das verbleibende Produkt kann nach üblichen Methoden, wie zum Beispiel Säulenchromatographie, von eventuell noch vorhandenen Verunreinigungen befreit werden.

Das bei dem erfindungsgemäßen Verfahren (a) als Reaktionskomponente benötigte Dimethyl-oxo-sulfonium-methylid der Formel (III) ist bekannt (vgl. J. Am. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, in Gegenwart eines Verdünnungsmittels erzeugt.

Das bei dem erfindungsgemäßen Verfahren (a) außerdem als Reaktionskomponente in Betracht kommende Dimethylsulfonium-methylid der Formel (IV) ist ebenfalls bekannt (vgl. Heterocycles 8, 397 (1977)). Es wird bei der obigen Umsetzung ebenfalls in frisch hergestelltem Zustand eingesetzt, indem man es in situ zum Beispiel aus Trimethylsulfonium-halogenid oder Trimethylsulfoniummethylsulfat, in Gegenwart einer starken Base, wie zum Beispiel Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Die bei dem erfindungsgemäßen Verfahren (a) als Zwischenprodukte auftretenden Oxirane der Formel (V) sind noch nicht bekannt. Sie stellen allgemein interessante Zwischenprodukte dar.

Die für die zweite Stufe des erfindungsgemäßen Verfahrens (a) als Reaktionskomponenten benötigten Azole der Formel (VI) sind allgemein bekannte Verbindungen der organischen Chemie.

Als Verdünnungsmittel kommen bei der Durchführung der ersten Stufe des erfindungsgemäßen Verfahrens (a) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei der Durchführung der ersten Stufe des erfindungsgemäßen Verfahrens in einem größeren Bereich variiert werden. Im allgemeinen arbeitet man zwischen 0 °C und 100 °C, vorzugsweise zwischen 10 °C und 60 °C.

Bei der Durchführung der ersten Stufe des erfindungsgemäßen Verfahrens (a) setzt man auf 1 Mol an Phenylcyclopropyl-keton der Formel (II) im allgemeinen 1 bis 3 Mol an Dimethyloxosulfonium-methylid der Formel (III) bzw. an Dimethylsulfonium-methylid der Formel (IV) ein. Die Isolierung der Oxirane der Formel (V) erfolgt nach üblichen Methoden.

Die zweite Stufe des erfindungsgemäßen Verfahrens (a) wird in Gegenwart einer Base durchgeführt. Dabei kommen alle üblicherweise verwendbaren anorganischen und organischen Basen in Betracht. Vorzugsweise verwendbar sind Alkalicarbonate, wie z.B. Natrium- und Kaliumcarbonat; Alkalihydroxide, wie z.B. Natriumhydroxid; Alkalialkoholate, wie z.B. Natrium- und Kaliummethylat und -ethylat; Alkalihydride, wie z.B. Natriumhydrid, sowie niedere tertiäre Alkylamine, Cycloalkylamine und Aralkylamine, wie insbesondere Triethylamin.

Als Verdünnungsmittel kommen für die zweite Stufe des erfindungsgemäßen Verfahrens (a) inerte organische Lösungsmittel in Frage. Vorzugsweise verwendbar sind Nitrile, wie insbesondere Acetonitril; aromatische Kohlenwasserstoffe, wie Benzol, Toluol und Dichlorbenzol; Formamide, wie insbesondere Dimethylformamid, sowie Hexamethylphosphorsäuretriamid.

Die Reaktionstemperaturen können bei der Durchführung der zweiten Stufen des erfindungsgemäßen Verfahrens (a) in einem größeren Bereich variiert werden. Im allgemeinen arbeitet man bei Temperaturen zwischen 0 °C und 200 °C, vorzugsweise zwischen 60 °C und 150 °C.

Bei der Durchführung der zweiten Stufe des erfindungsgemäßen Verfahrens (a) setzt man vorzugsweise auf 1 Mol an Oxiran der Formel (V), 1 bis 2 Mol an Azol der Formel (VI) und 1 bis 2 Mol an Base ein. Die Isolierung der Endprodukte erfolgt in üblicher Weise.

Die für das erfindungsgemäße Verfahren (b) als Ausgangsstoffe benötigten Azolylmethyl-cyclopropyl-carbinol-Derivate der Formel (Ia) sind erfindungsgemäße Verbindungen.

Als Reaktionskomponenten kommen bei dem erfindungsgemäßen Verfahren (b) alle für derartige Umsetzungen übliche Oxidationsmittel in Betracht. Vorzugsweise verwendbar sind Wasserstoffperoxid und Persäuren, wie m-Chlorperbenzoesäure und Peressigsäure.

Bei der Durchführung des erfindungsgemäßen Verfahrens (b) setzt man auf 1 Mol der erfindungsgemäßen Verbindungen der Formel (Ia) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Essigsäure oder Acetanhydrid bei Temperaturen zwischen -30 °C bis +90 °C, entstehen vorzugsweise die erfindungsgemäßen Verbindungen der Formel (I) mit der -SO-Gruppierung. Bei Überschuß an Oxidationsmittel und Temperaturen zwischen 10 °C und 90 °C entstehen vorzugsweise die erfindungsgemäßen Verbindungen der Formel (I) mit einer -$SO_2$-Gruppierung. Die Isolierung der Oxidationsprodukte erfolgt in üblicher Weise.

Die für das erfindungsgemäße Verfahren (c) als Ausgangsstoffe benötigten Azolylmethyl-cyclopropylcarbinol-Derivate der Formel (Ib) sind ebenfalls erfindungsgemäße Verbindungen. Ihre Überführung in die entsprechenden Alkoholate erfolgt in allgemein bekannter Weise, indem man mit geeigneten starken Basen, wie Alkalimetall-ami den oder -hydriden, quarternären Ammonium-hydroxiden oder Phosphonium-hydroxiden in einem inerten Verdünnungsmittel, wie zum Beispiel Dioxan, bei Raumtemperatur umsetzt. Demgemäß steht Y in den Verbindungen der Formel (Ic) vorzugsweise für ein Alkalimetallkation, wie ein Natrium- oder Kaliumkation, oder für ein quarternäres Ammonium- oder Phosphoniumkation.

Die bei dem erfindungsgemäßen Verfahren (c) außerdem als Ausgangsstoffe benötigten Halogenverbindungen sind durch die Formel (VII) allgemein definiert. In dieser Formel steht $R^2$ vorzugsweise für die Bedeutungen, die bereits im Zusammenhang mit der Beschreibung der erfindungsgemäßen Stoffe der Formel (I) für den Substituenten $R^1$ genannt wurden, mit Ausnahme der Bedeutung von Wasserstoff. Hal steht vorzugsweise für Chlor oder Brom.

Die Halogenverbindungen der Formel (VII) sind bekannt oder lassen sich nach im Prinzip bekannten Methoden herstellen.

Als Verdünnungsmittel kommen bei der Durchführung des erfindungsgemäßen Verfahrens (c) inerte organische Lösungsmittel in Frage. Hierzu gehören vorzugsweise Ether, wie Diethylether oder Dioxan; aromatische Kohlenwasserstoffe, wie Benzol; in einzelnen Fällen auch chlorierte Kohlenwasserstoffe, wie Chloroform, Methylenchlorid oder Tetrachlorkohlenstoff; sowie Hexamethylphosphorsäuretriamid.

Die Reaktionstemperaturen können bei der Durchführung des erfindungsgemäßen Verfahrens (c) in einem größeren Bereich variiert werden. Im allgemeinen arbeitet man zwischen 0 °C und 120 °C, vorzugsweise zwischen 20 °C und 100 °C.

Bei der Durchführung des erfindungsgemäßen Verfahrens (c) setzt man zunächst Hydroxy-Verbindungen der Formel (Ib) mit starken Basen zu den entsprechenden Alkoholaten der Formel (Ic) um. In der danach folgenden Stufe setzt man auf 1 Mol eines Alkoholates der Formel (Ic) vorzugsweise 1 bis 2 Mol Halogenverbindung der Formel (VII) ein.

Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform wird zweckmäßigerweise so verfahren, daß man von einer Hydroxy-Verbindung der Formel (Ib) ausgeht, letztere in einem geeigneten organischen Lösungsmittel mittels Alkalimetall-hydrid oder Alkalimetall-amid in das Alkalimetallalkoholat überführt und letzteres ohne Isolierung sofort mit einer Halogenverbindung der Formel (VII) umsetzt, wobei unter Austritt von Alkalimetallhalogenid die erfindungsgemäßen Verbindung der Formel (I) in einem Arbeitsgang erhalten werden.

Nach einer weiteren bevorzugten Ausführungsform werden zweckmäßigerweise die Herstellung der Alkohole sowie die Umsetzung mit einer Halogenverbindung der Formel (VII) in einem Zweiphasensystem, wie beispielsweise wäßrige Natron- oder Kalilauge/Toluol oder Methylenchlorid, unter Zusatz von 0,01-1 Mol eines Phasen-Transfer-Katalysators, wie beispielsweise Ammonium- oder Phosphoniumverbindungen, durchgeführt, wobei in der organischen Phase oder an der Grenzfläche die Alkoholate mit den in der organischen Phase befindlichen Halogeniden umgesetzt werden.

Die nach den erfindungsgemäßen Verfahren erhältlichen Azolylmethyl-cyclopropyl-carbinol-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phythophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;

(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Venturia im Obstbau, zur Bekämpfung von Erysiphe, Leptosphaeria, Pseudocercosporella und Fusarium im Getreidebau, sowie zur Bekämpfung von Pyricularia an Reis. Die erfindungsgemäßen Stoffe zeigen auch eine breite in-vitro-Wirkung.

Außerdem besitzen die erfindungsgemäßen Wirkstoffe auch pflanzenwuchsregulierende Eigenschaften.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstrumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstrumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten-und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstrumsregulatoren kann es zur Ausbildung parthenokarper Früchte kommen. Ferner kann das Geschlecht der Blüten beeinflußt werden. Auch kann eine Sterilität der Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstrumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Tixanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des

Bodens sind Wirkstoffkonzentrationen von 0,0001 bis 0.1 Gew.-%, vorzugsweise von 0,0001 bis 0,02 %, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele

Beispiel 1

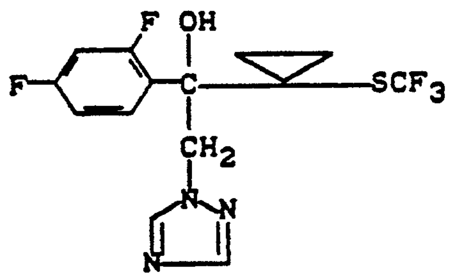

(I-1)

In ein Gemisch aus 12 g (174 mMol) 1,2,4-Triazol und 1,4 g (12,5 mMol) Kalium-tert.-butylat in 30 ml absolutem Dimethylformamid wird unter Stickstoffatmosphäre und unter Rühren eine Lösung von 15,9 g (54 mMol) 2-(2,4-Difluorphenyl)-2-[1-(trifluormethylmercapto)-cycloprop-1-yl]-oxiran in 20 ml absolutem Dimethylformamid eingetropft. Das Reaktionsgemisch wird 8 Stunden bei 100° C gerührt. Danach zieht man das Lösungsmittel unter vermindertem Druck ab, nimmt den Rückstand in Essigsäureethylester auf, wäscht zweimal mit Wasser und engt nach dem Trocknen über Natriumsulfat unter vermindertem Druck ein. Der Rückstand wird mit Chloroform als Laufmittel über eine Kieselgelsäule chromatographiert. Man erhält auf diese Weise 12,5 g (65 % der Theorie) an 1-(2,4-Difluorphenyl)-1-hydroxy-2-(1,2,4-triazol-1-yl)-1-(1-trifluormethylmercapto-cycloprop-1-yl)-ethan vom Schmelzpunkt 103° C.

Herstellung von Ausgangsprodukten

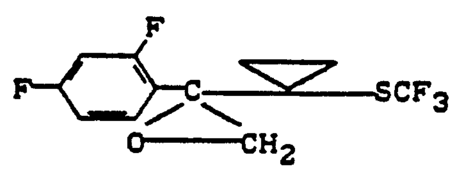

(V-1)

In ein Gemisch von 1,9 g (63 mMol) Natriumhydrid (80 %ig) und 13,5 g (61 mMol) Trimethyloxosulfonium-iodid werden bei 10° C unter Stickstoffatmosphäre 50 ml absolutes Dimethylsulfoxid getropft. Das Gemisch wird noch eine Stunde bei 10° C gerührt und dann bei der gleichen Temperatur tropfenweise mit einer Lösung von 15,3 g (54 mMol) 2,4-Difluorphenyl-(1-trifluormethylmercapto-cycloprop-1-yl)-keton in 20 ml abolutem Dimethylsulfoxid versetzt. Man rührt 48 Stunden bei Raumtemperatur und anschließend 1 Stunde bei 40° C. Das Reaktionsgemisch wird auf Wasser gegossen. Man extrahiert mit

Essigsäureethylester, wäscht die vereinigten organischen Phasen mit Wasser, trocknet über Natriumsulfat und engt unter vermindertem Druck ein. Man erhält (15,9 g (99 % der Theorie) an 2-(2,4-Difluorphenyl)-2-[1-(trifluormethylmercapto)-cycloprop-1-yl]-oxiran in Form eines farblosen Öles.
$^{1}$H-NMR (360 MHz; $CDCl_3$):
$\delta$ = 1,00-1,50 (m, 4H); 3,04 (m,2H); 6,80-6,96 (m, 2H); 7,38-7,48 (m,1H).

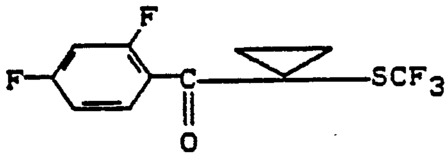

(II-1)

Eine Lösung von 47 g (0,15 Mol) (3-Chlor-1-trifluormethylmercapto-propyl)-(2,4-difluorphenyl)-keton in 70 ml tert.-Butanol wird bei 40 °C unter Rühren in ein Gemisch aus 20 g (0,18 Mol) Kalium-tert.-butylat und 50 ml tert.-Butanol getropft. Das Reaktionsgemisch wird 4 Stunden bei 40 °C gerührt und dann auf Wasser gegossen. Man extrahiert mit Essigsäureethylester, wäscht die vereinigten organischen Phasen mit Wasser, trocknet über Natriumsulfat und engt durch Abziehen des Lösungsmittels unter vermindertem Druck ein. Der verbleibende Rückstand wird über eine Kieselgelsäule mit Petrolether: Methylenchlorid = 1:0,4 als Laufmittel chromatographiert. Man erhält 15,3 g (36 % der Theorie) 2,4-Difluorphenyl-(1-trifluormethylmercapto-cycloprop-1-yl)-keton in Form eines farblosen Öles.
$^{1}$H-NMR (200 MHz, $CDCl_3$):
$\delta$ = 1,57 (m, 2H); 1,95 (m, 2H); 6,80-7,02 (m, 2H); 7,55-7,68 (m, 1H).

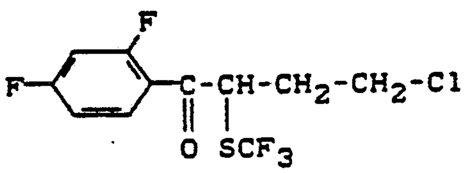

(X-1)

In eine Lösung von 43 g (0,2 Mol) 3-Chlorpropyl-2,4-difluorphenyl-keton in 100 ml Methylenchlorid werden bei 20 bis 25 °C unter Rühren 75 g (0,55 Mol) Trifluormethylsulfenylchlorid gegeben. Das Gemisch wird 108 Stunden bei Raumtemperatur gerührt und dann durch Abziehen des Lösungsmittels unter vermindertem Druck eingeengt. Man erhält 63 g (3-Chlor-1-trifluormethylmercapto-propyl)-2,4-difluorphenyl-keton.
$^{19}$F-NMR (Externer Standard: $CF_3COOH$):
$\delta$ = - 36,6 ppm (Duplett)
+ 24,5 ppm (Multiplett)
+ 27,9 ppm (Multiplett)

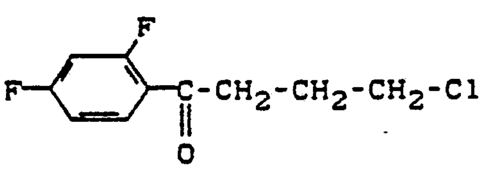

(VIII-1)

In ein Gemisch von 50 g (0,44 Mol) 1,3-Difluorbenzol und 64 g (0,48 Mol) wasserfreiem Aluminiumchlorid werden bei 20 °C unter Rühren 62 g (0,44 Mol) 4-Chlorbuttersäurechlorid getropft. Das Reaktionsgemisch wird 3,5-Stunden bei 30 °C gerührt bis eine klare Lösung entstanden ist und die Gasentwicklung nachgelassen hat. Nach dem Abkühlen auf Raumtemperatur werden 300 ml Methylenchlorid zugegeben. Man gießt die entstandene Lösung auf 300 g Eis, trennt die organische Phase ab, trocknet über Natriumsulfat und engt durch Abziehen des Lösungsmittels unter vermindertem Druck ein. Man erhält 83 g (86 % der Theorie) an 3-Chlorpropyl-2,4-difluorphenyl-keton in Form einer Flüssigkeit.
Kp = 80 °C · 0,1 mbar

Beispiel 2

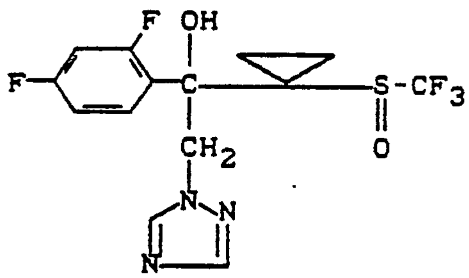

(I-2)

In eine Lösung von 4 g 1-(2,4-Difluorphenyl)-1-hydroxy-2-(1,2,4-triazol-1-yl)-1-(1-trifluormethylmercapto-cycloprop-1-yl)-ethan in 30 ml Eisessig werden bei 85° C unter Rühren 6 ml 35 %ige Wasserstoffperoxid-Lösung eingetropft. Das Reaktionsgemisch wird 4 Stunden bei 85° C gerührt und dann auf 80 ml Wasser gegossen. Durch Zugabe von verdünnter, wäßriger Natronlauge wird die Lösung basisch gestellt. Man extrahiert mehrfach mit Methylenchlorid, wäscht die vereinigten organischen Phasen mit verdünnter, wäßriger Natronlauge und Wasser, trocknet dann über Natriumsulfat und engt durch Abziehen des Lösungsmittels unter vermindertem Druck ein. Der verbleibende Rückstand wird an Kieselgel chromatographisch gereinigt. Man erhält 1,7 g (41 % der Theorie) an 1-(2,4-Difluorphenyl)-1-hydroxy-2-(1,2,4-triazol-1-yl)-1-(trifluormethylsulfoxy-cycloprop-1-yl)-ethan vom Schmelzpunkt 101° C.

Nach der im Beispiel 1 angegebenen Methode wurden auch die in den folgenden Beispielen aufgeführten Verbindungen hergestellt:

**Beispiel 3**

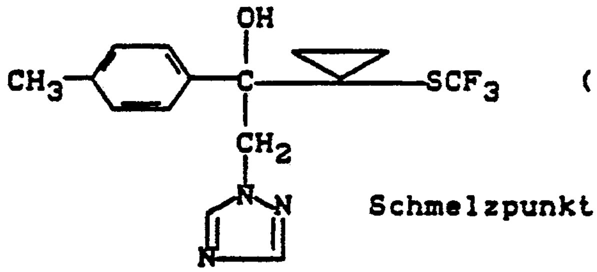

(I-3)

Schmelzpunkt 127° C

**Beispiel 4**

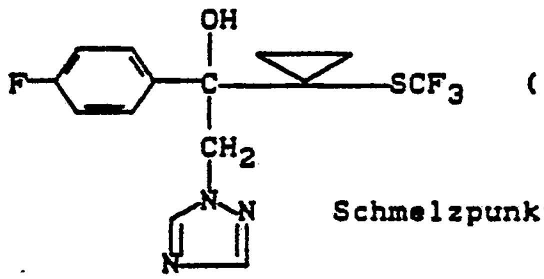

(I-4)

Schmelzpunkt 128° C

**Beispiel 5**

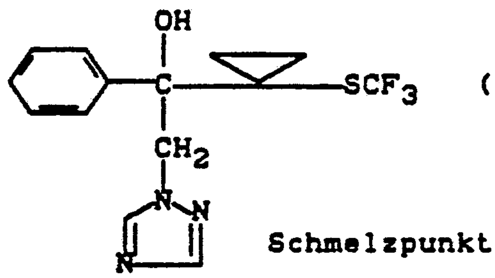

(I-5)

Schmelzpunkt 124° C

In den folgenden Verwendungsbeispielen wurde die nachstehend aufgeführte Verbindung als Vergleichssubstanz eingesetzt:

(A) = 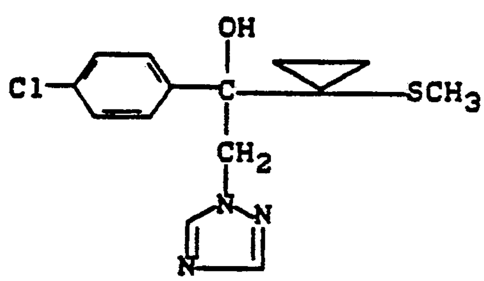

(bekannt aus EP-OS 0 180 136)

Beispiel A

Fusarium culmorum-Test (Weizen) Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 x 100 Korn 1 cm tief in eine Standarderde und kultiviert ihn im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome.

In diesem Test zeigt die erfindungsgemäße Verbindung (I-1) eine wesentlich bessere Wirksamkeit als die Vergleichssubstanz (A).

Beispiel B

Cercospora-Test (Mungobohne) protektiv

Lösungsmittel: 4,7 Gewichtsteile Aceton

Emulgator: 0,3 Gewichtsteile Alkyl-arylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Cercospora canescens inokuliert und verbleiben einen Tag in einer dunklen Feuchtekammer bei 22°C und 100 % relativer Luftfeuchtigkeit.

Anschließend werden die Pflanzen unter Belichtung im Gewächshaus bei 23°C und 80% relativer Luftfeuchtigkeit aufgestellt.

Etwa 20 Tage nach der Inokulation erfolgt die Auswertung.

In diesem Test zeigt die erfindungsgemäße Verbindung (I-1) eine bessere Wirksamkeit als die Vergleichssubstanz (A).

## Ansprüche

1. Azolylmethyl-cyclopropyl-carbinol-Derivate der Formel

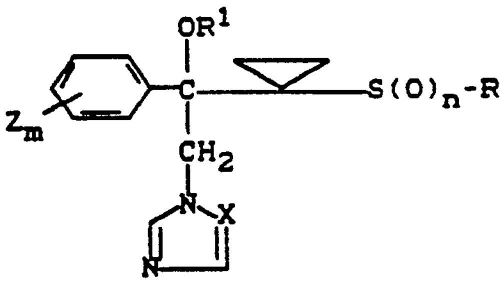

(I)

in welcher

R für Perfluoralkyl mit 1 bis 4 Kohlenstoffatomen, Trichlormethyl, Difluorchlormethyl oder Fluordichlormethyl steht,

R' für Wasserstoff, Alkyl oder Acyl steht,

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,

m für die Zahlen 0, 1, 2 oder 3 steht und

n für die Zahlen 0, 1 oder 2 steht und

X für Stickstoff oder eine CH-Gruppe steht,

sowie deren Säureadditions-Salze und Metallsalz-Komplexe.

2. Azolylmethyl-cyclopropyl-carbinol-Derivate der Formel (I) gemäß Anspruch 1, in denen

R für Trifluormethyl, Trichlormethyl, Difluorchlormethyl oder Fluordichlormethyl steht,

R' für Wasserstoff, Methyl, Ethyl, n-Propyl, Isopropyl, n-Butyl, Isobutyl, Methylcarbonyl, Ethylcarbonyl, n-Propylcarbonyl, Isopropylcarbonyl, n-Butylcarbonyl und Isobutylcarbonyl steht,

Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy steht,

m für die Zahlen 0, 1, 2 oder 3 steht,

n für die Zahlen 0, 1 oder 2 steht und

X für Stickstoff oder eine CH-Gruppe steht.

3. Verfahren zur Herstellung von Azolylmethyl-cyclopropyl-carbinol-Derivaten der Formel

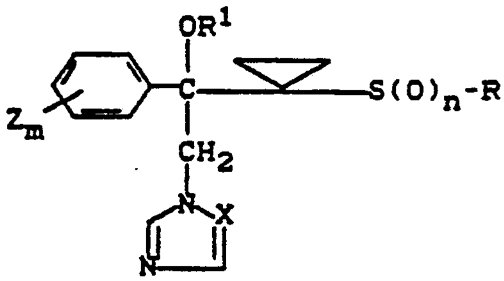

(I)

in welcher

R für Perfluoralkyl mit 1 bis 4 Kohlenstoffatomen, Trichlormethyl, Difluorchlormethyl oder Fluordichlormethyl steht,

R' für Wasserstoff, Alkyl oder Acyl steht,

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,

m für die Zahlen 0, 1, 2 oder 3 steht und

n für die Zahlen 0, 1 oder 2 steht und

X für Stickstoff oder eine CH-Gruppe steht,

sowie von deren Säureadditions-Salzen und Metallsalz-Komplexen, dadurch gekennzeichnet, daß man

a) in einer <u>ersten Stufe</u> Phenyl-cyclopropylketone der Formel

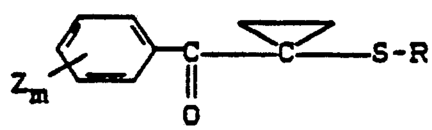

(II)

in welcher
R, Z und m die oben angegebene Bedeutung haben,
entweder
α) mit Dimethyloxosulfonium-methylid der Formel

$$(CH_3)_2\overset{\delta\oplus}{S}O\overset{\delta\ominus}{C}H_2 \qquad (III)$$

oder
β) mit Dimethylsulfonium-methylid der Formel

$$(CH_3)_2\overset{\delta\oplus}{S}\ \overset{\delta\ominus}{C}H_2 \qquad (IV)$$

in Gegenwart eines Verdünnungsmittels umsetzt und
in einer <u>zweiten Stufe</u> die dabei erhaltenen Oxirane der Formel

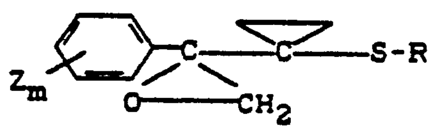

(V)

in welcher
R, Z und m die oben angegebene Bedeutung haben,
mit Azolen der Formel

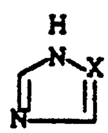

(VI)

in welcher
X die oben angegebene Bedeutung hat,
in Gegenwart eines Säurebindemittels und in Gegenwart eines Verdünnungsmittels umsetzt,
oder
b) Azolylmethyl-cyclopropyl-carbinol-Derivate der Formel

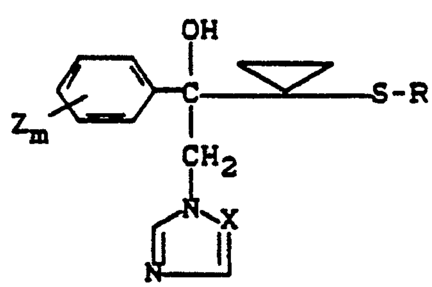

(Ia)

in welcher

R, X, Z und m die oben angegebene Bedeutung haben,
mit Oxidationsmitteln gegebenenfalls in Gegenwart eines Verdünnungsmittels umsetzt,
oder
c) Azolylmethyl-cyclopropyl-carbinol-Derivate der Formel

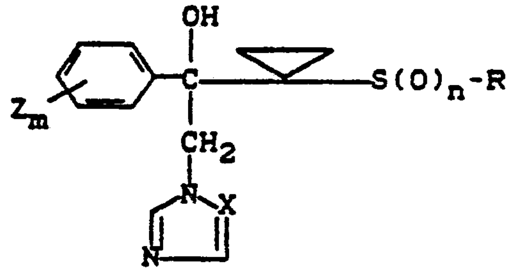

(Ib)

in welcher
R, Z, X, m und n die oben angegebene Bedeutung haben,
mit starken Basen in Gegenwart eines Verdünnungsmittels umsetzt und die dabei entstehenden Alkoholate der Formel

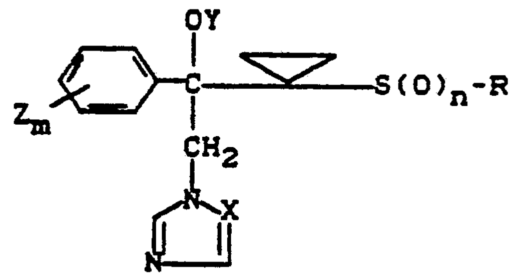

(Ic)

in welcher
R, X, Z, m und n die oben angegebene Bedeutung haben und
Y für einen kationischen Rest einer Base steht,
mit Halogenverbindungen der Formel
$R^2$-Hal (VII)
in welcher
$R^2$ für Alkyl oder Acyl steht und
Hal für Halogen steht,
in Gegenwart eines Verdünnungsmittels umsetzt,
und gegebenenfalls anschließend an die so erhaltenen Verbindungen der Formel (I) eine Säure oder ein Metallsalz addiert.

4. Fungizide und pflanzenwuchsregulierende Mittel, gekennzeichnet durch einen Gehalt an mindestens einem Azolylmethyl-cyclopropyl-carbinol-Derivat der Formel (I) gemäß Anspruch 1 bzw. an einem Säureadditions-Salz oder Metallsalz-Komplex eines Azolylmethyl-cyclopropyl-carbinol-Derivates der Formel (I).

5. Verwendung von Azolylmethyl-cyclopropyl-carbinol-Derivaten der Formel (I) gemäß Anspruch 1 bzw. von deren Säureadditions-Salzen und Metallsalz-Komplexen zur Bekämpfung von Pilzen sowie zur Regulierung des Pflanzenwachstums.

6. Verfahren zur Bekämpfung von Pilzen und zur Regulierung des Pflanzenwachstums, dadurch gekennzeichnet, daß man Azolylmethyl-cyclopropyl-carbinol-Derivate der Formel (I) gemäß Anspruch 1 bzw. deren Säureadditions-Salze oder Metallsalz-Komplexe auf die Pflanzen und/oder deren Lebensraum ausbringt.

7. Verfahren zur Herstellung von fungiziden und pflanzenwuchsregulierenden Mitteln, dadurch gekennzeichnet, daß man Azolylmethyl-cyclopropyl-carbinol-Derivate der Formel (I) gemäß Anspruch 1 bzw. deren Säureadditionssalze oder Metallsalz-Komplexe mit Streckmitteln und/oder oberflächenaktiven Stoffen vermischt.

8. Phenyl-cyclopropyl-ketone der Formel

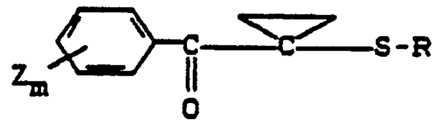

(II)

in welcher
R für Perfluoralkyl mit 1 bis 4 Kohlenstoffatomen, Trichlormethyl, Difluorchlormethyl oder Fluordichlormethyl steht,
Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen,
Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und
m für die Zahlen 0, 1, 2 oder 3 steht.

9. Verfahren zur Herstellung von Phenyl-cyclopropylketonen der Formel

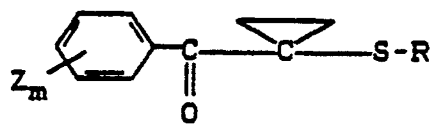

(II)

in welcher
R für Perfluoralkyl mit 1 bis 4 Kohlenstoffatomen, Trichlormethyl, Difluorchlormethyl oder Fluordichlormethyl steht,
Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und
m für die Zahlen 0, 1, 2 oder 3 steht,
dadurch gekennzeichnet, daß man
d) in einer ersten Stufe Phenylketone der Formel

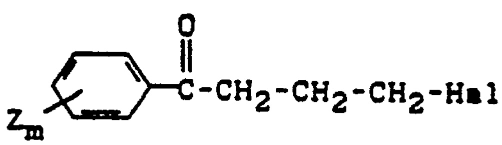

(VIII)

in welcher
Z und m die oben angegebene Bedeutung haben und
Hal für Chlor oder Brom steht,
mit Mercaptanen der Formel
Hal′-S-R (IX)
in welcher
R die oben angegebene Bedeutung hat und
Hal′ für Chlor oder Brom steht,
in Gegenwart eines Verdünnungsmittels umsetzt und die entstehenden Phenylpropyl-ketone der Formel

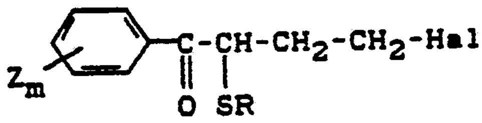

(X)

in welcher
R, Z, Hal und m die oben angegebene Bedeutung haben,
in einer zweiten Stufe in Gegenwart eines Säurebindemittels und in Gegenwart eines Verdünnungsmittels umsetzt.

10. Oxirane der Formel

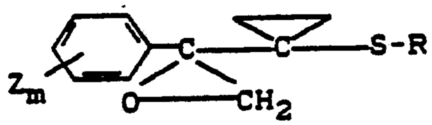

(V)

in welcher
R für Perfluoralkyl mit 1 bis 4 Kohlenstoffatomen, Trichlormethyl, Difluorchlormethyl oder Fluordichlormethyl steht,
Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und
m für die Zahlen 0, 1, 2 oder 3 steht.

Europäisches Patentamt

# EUROPÄISCHER RECHERCHENBERICHT

Nummer der Anmeldung

EP 88 10 9423

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | DE-A-3 617 190 (SANDOZ-PATENT GMBH) * ganzes Dokument, besonders Seite 5, Zeilen 8,9,12; Seite 6, Zeilen 1-3,5,6 * | 1,3-10 | C 07 D 249/08<br>C 07 D 233/60<br>C 07 D 303/34<br>C 07 C 149/267<br>C 07 C 149/273<br>A 01 N 43/50<br>A 01 N 43/653 |
| A | EP-A-0 086 173 (CIBA-GEIGY AG) * Seiten 1-4, besonders Seite 2, Zeilen 2,3,6; Seiten 9,10 * | 1,3-7, 10 | |
| A,D | EP-A-0 180 136 (BAYER AG) * Ansprüche 1-7 * | 1,3-10 | |
| | | | **RECHERCHIERTE SACHGEBIETE (Int. Cl.4)** |
| | | | C 07 D 249/00<br>C 07 D 233/00<br>C 07 D 521/00<br>C 07 D 303/00<br>C 07 C 149/00<br>A 01 N 43/00 |

Der vorliegende Recherchenbericht wurde für alle Patentansprüche erstellt

| Recherchenort | Abschlußdatum der Recherche | Prüfer |
|---|---|---|
| BERLIN | 12-09-1988 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)